# NEUE EUROPÄISCHE PATENTSCHRIFT

(11) **EP 2 346 982 B2**
(45) Veröffentlichungstag und Bekanntmachung des Hinweises auf die Entscheidung über den Einspruch: **13.03.2019**
(45) Hinweis auf die Patenterteilung: 20.03.2013
(21) Anmeldenummer: 09760730.3
(22) Anmeldetag: 13.11.2009
(51) Int. Cl.: C12M 1/107

(54) **VORRICHTUNG UND VERFAHREN ZUR REGELUNG DER GASZUFUHR BZW. DES GASTRANSPORTS BEI EINEM GASSPEICHERSYSTEM**
DEVICE AND METHOD FOR REGULATING THE GAS SUPPLY OR THE GAS TRANSPORT IN A GAS STORAGE SYSTEM
DISPOSITIF ET PROCÉDÉ DE RÉGULATION D'APPORT DE GAZ OU DE TRANSPORT DE GAZ DANS UN SYSTÈME DE STOCKAGE DE GAZ

(30) Priorität: 15.11.2008 DE 102008057586
(43) Veröffentlichungstag der Anmeldung: 27.07.2011
(73) Patentinhaber: MT-Energie Service GmbH, 27404 Zeven (DE)
(72) Erfinder: MARTENS, Christoph, 27404 Rockstedt (DE); BEHRENS, Jan, C. F., 27404 Seedorf (DE)
(74) Vertreter: Hauck Patentanwaltspartnerschaft mbB
(86) Internationale Anmeldenummer: PCT/EP2009/008088
(87) Internationale Veröffentlichungsnummer: WO 2010/054827

(56) Entgegenhaltungen:
- EP-A2- 0 350 455
- EP-A2- 1 338 843
- EP-A2- 1 447 613
- AT-B- 388 158
- DE-C2- 3 903 891
- DE-C2- 4 244 612
- DE-T2- 68 922 151
- DE-U1-202004 020 394
- DE-U1-202004 020 394
- US-B2- 6 810 925

## Beschreibung

Die Erfindung betrifft eine Vorrichtung und ein Verfahren zur Regelung der Gaszufuhr bzw. des Gastransports bei einem Gasspeichersystem einer Biogasanlage, wobei das Gasspeichersystem mindestens zwei kaskadenartig in Reihe geschaltete Gasspeicher aufweist.

Bekannte Biogasanlagen weisen oftmals mehrere Gasspeicher in Form von abgedeckten Gärbehältern auf, die kaskadenartig hintereinander geschaltet in einem Gasverbundsystem stehen. Die Behälter sind häufig mit einer Tragluftfolienabdeckung versehen, die eine Doppelmembranabdeckung bildet. Dabei wird eine Gasspeichermembran, beispielsweise eine Gasspeicherfolie über der oberen Öffnung und dem Rand des Gärbehälters installiert. Zusätzlich wird über der Gasspeicherfolie eine Schutzabdeckung, beispielsweise eine Wetterschutzfolie, installiert. Die Gasspeicherfolie und die Wetterschutzfolie werden gasdicht und kraftschlüssig mit dem Wandkopf des Gärbehälters verbunden. Es ist bekannt, in den Zwischenraum, der sich zwischen Gasspeicherfolie und Wetterschutzfolie bildet, mittels eines Stützluftgebläses Luft einzuleiten. Auf diese Weise werden die Formstabilität der Wetterschutzfolie gewährleistet und eine unerwünschte Beeinflussung der Formstabilität durch beispielsweise Schnee oder Regenwasser vermieden. Über mindestens eine ebenfalls an dem Zwischenraum vorgesehene druckregelnde Entlüftungsöffnung, beispielsweise eine Querstromklappe, kann die eingeleitete Luft wieder entweichen. Die Klappe wird mit Gewichten beaufschlagt, die den Staudruck des Auslasses regulieren und somit den Systemdruck für den jeweiligen Gasspeicher erzeugen. Unter der formstabilen Wetterschutzfolie kann sich die Gasspeicherfolie weitgehend frei auf und ab bewegen und so das Gasspeichervolumen in dem Behälter der Biogasanlage zur Verfügung stellen.

Das in Gärbehältern, beispielsweise Fermentern, von Biogasanlagen produzierte Gas wird üblicherweise mittels einer biologischen Entschwefelung gereinigt. Für den biologischen Abbau von Schwefelwasserstoff (H₂S) in der Gasphase sind sogenannte Thiobazillen verantwortlich, die natürlich vorhanden sind. Thiobazillen setzen H₂S auf aerobem Weg zu Schwefelsäure und elementarem Schwefel um. Die Reaktionsgleichungen sind im Folgenden dargestellt:

1) H₂S + ½O₂ → S + H₂O 2) H₂S + 2 O₂ → H₂SO₄

Für die Entschwefelung muss ausreichend Sauerstoff in der Gasphase vorhanden sein. Hierzu wird Luft bzw. Sauerstoff in definierten Mengen in den Gärbehälter über der jeweiligen Gärsubstanz eingeleitet. Darüber hinaus muss eine ausreichend große Besiedelungsfläche für die Mikroorganismen vorhanden sein (z.B. Netz). Die Güte der Gasreinigung ist u.a. abhängig von der Verweilzeit des Gases in der Biogasanlage. Bei nicht beheizten Gärproduktlagern in der Biogasanlage kann sich das Gas beim Durchleiten durch die kaskadenartig angeordneten Behälter abkühlen und somit einen Teil der im Gas enthaltenen Feuchtigkeit abgeben.

Es ist bekannt, mehrere Gasspeicher in Serie zu verschalten, wobei zwischen den Gasspeichern Gasleitungen verlaufen, durch die das Gas transportiert wird. Da der Gastransport insbesondere ohne Gasverdichter erfolgen kann, muss von einem Gasspeicher zum nächsten der Druck abnehmen. In den Gasleitungen entstehen weitere Druckverluste. Im ersten Gasspeicher herrscht ein Startdruck, der aufgrund der baulichen Gegebenheiten, insbesondere der statischen Auslegung der Folienabdeckung, einen vorgegebenen Maximalwert nicht überschreiten darf. Im letzten Gasspeicher der Anlage sollte noch ein Mindestdruck herrschen, damit das Gas aus diesem Gasspeicher problemlos entnommen werden kann.

Es besteht somit die Notwendigkeit, die Drücke in den einzelnen Gasspeichern zu regulieren. Der über der Gärsubstanz herrschende Gärdruck kann dabei durch eine Steuerung des in dem Zwischenraum zwischen Gasspeicherfolie und Wetterschutzfolie herrschenden Drucks beeinflusst werden, wobei die Gasspeicherfolie als Membran wirkt. Bei einer Kaskadenschaltung der Gasspeicherbehälter werden die Behälterdrücke üblicherweise über die auf die jeweiligen Entlüftungsklappen wirkenden Gewichte reguliert. Das Stützluftgebläse wird dabei kontinuierlich betrieben.

In der Praxis wird versucht, bei der Inbetriebnahme der Anlage ein Gleichwicht im System herzustellen, so dass das Gas der Reihe nach durch die Gasspeicher fließt. Ändern sich nach der Anlaufphase jedoch der Zustand und die Einflüsse auf das System (z.B. durch geänderte Gasproduktionsmengen oder jahreszeitliche Klimawechsel) kann die Anlage nicht selbständig auf Veränderungen reagieren. Es kann zu Störungen des Gasflusses kommen. Um die sensible Abstimmung der Gasdrücke anzupassen, ist es daher oftmals erforderlich, dass ein Betreiber vor Ort über die Gewichtsbeaufschlagung der Entlüftungsklappen Eingriff nimmt. Dies ist mit einem erheblichen Aufwand verbunden. Außerdem ist eine flexible Reaktion auf geänderte Betriebszustände ohne aufwändigen Eingriff durch Personal vor Ort kaum möglich. Dies gilt insbesondere, wenn Gasspeicher für Wartungs- oder Reparaturzwecke in kurzer Zeit vollständig entleert werden sollen.

Hinzukommt, dass die Gewichte nicht immer eine ausreichend feine Druckeinstellung erlauben. Insbesondere bei größeren Kaskadenschaltungen, beispielsweise von mehr als vier Behältern, müssen aufgrund des vorgegebenen maximalen Startdrucks im ersten Behälter und des ebenfalls vorgegebenen Mindestdrucks im letzten Behälter teilweise äußerst geringe Differenzdrücke zwischen den Behältern eingestellt werden. Derart geringe Druckunterschiede sind insbesondere aufgrund der verhältnismäßig groben Einstellungsmöglichkeiten durch Gewichte nicht immer praxistauglich realisierbar.

Ausgehend von dem erläuterten Stand der Technik liegt der Erfindung die Aufgabe zugrunde, eine Vorrichtung und ein Verfahren der eingangs genannten Art bereitzustellen, mit denen in einfacher, flexibler und präziser Weise ein Gasspeichermanagement ermöglicht wird.

Die Erfindung löst diese Aufgabe gemäß einem ersten Aspekt durch die Gegenstände der Ansprüche 1 und 10. Nach einem zweiten Aspekt löst die Erfindung die Aufgabe durch die Gegenstände der Ansprüche 3 und 12. Vorteilhafte Ausgestaltungen finden sich in den abhängigen Ansprüchen, der Beschreibung sowie den Figuren.

Die Erfindung löst die Aufgabe für eine Vorrichtung der eingangs genannten Art gemäß einem ersten Aspekt dadurch, dass die Gasspeicher jeweils einen Zwischenraum zwischen einer Gasspeichermembran und einer Schutzabdeckung besitzen und dass die Vorrichtung Gaszuführeinrichungen, Zwischenraumdruckmesseinrichtungen und mindestens eine Regeleinrichtung umfasst, wobei jedem Gasspeicher jeweils mindestens eine Gaszuführeinrichung zugeordnet ist, mit der dem Zwischenraum des jeweiligen Gasspeichers Gas zugeführt werden kann, wobei jedem Gasspeicher jeweils mindestens eine Zwischenraumdruckmesseinrichtung zugeordnet ist, mit der der Gasdruck in dem Zwischenraum des jeweiligen Gasspeichers gemessen werden kann, wobei die Messwerte der Zwischenraumdruckmesseinrichtungen an der mindestens einen Regeleinrichtung anliegen, und wobei die mindestens eine Regeleinrichtung dazu ausgebildet ist, auf Grundlage der Messwerte der Zwischenraumdruckmesseinrichtungen den Gasdruck in den Zwischenräumen der Gasspeicher durch eine Ansteuerung der den Gasspeichern jeweils zugeordneten Gaszuführeinrichtungen zu regeln.

Die Erfindung löst die Aufgabe außerdem gemäß einem ersten Aspekt durch ein Verfahren der eingangs genannten Art, umfassend die Schritte: den Zwischenräumen der Gasspeicher wird jeweils Gas zugeführt, der Gasdruck in den Zwischenräumen wird jeweils gemessen, und auf Grundlage des gemessenen Gasdrucks in den Zwischenräumen wird jeweils durch eine Ansteuerung der Gaszufuhr in die Zwischenräume der Gasdruck in den Zwischenräumen geregelt.

Gemäß einem zweiten Aspekt löst die Erfindung die Aufgabe für eine Vorrichtung der eingangs genannten Art durch Anspruch 3.

Außerdem löst die Erfindung die Aufgabe für ein Verfahren der eingangs genannten Art durch Anspruch 12.

Auch die Gasspeicher nach dem zweiten Aspekt der Erfindung besitzen jeweils einen Zwischenraum zwischen einer Gasspeichermembran und einer Schutzabdeckung. Gemäß dem ersten Aspekt der Erfindung findet eine Regelung des Gasdrucks in dem Zwischenraum zwischen der Gasspeichermembran und der Schutzabdeckung jedes Gasspeichers statt. Gemäß dem zweiten Aspekt der Erfindung findet eine Regelung der Leistung des mindestens einen Gasverdichters statt. Die Gasspeichermembran kann eine Gasspeicherfolie sein und die Schutzabdeckung eine Schutzfolie, insbesondere eine Wetterschutzfolie. Es sind aber selbstverständlich auch feste Schutzabdeckungen denkbar. Die Gasspeicher können abgedeckte Gärbehälter der Biogasanlage sein, in denen entsprechend eine Gärsubstanz enthalten sein kann. Es kann sich aber auch um Gasspeicher handeln, in denen keine Gärsubstanz enthalten ist. Die Regelgröße ist nach dem ersten Aspekt der Erfindung der gemessene Gasdruck in den Zwischenräumen. Die Stellgröße ist die Gaszufuhr über die jeweilige Gaszuführeinrichtung. Die Regelung bzw. die Regeleinrichtung können nach dem ersten und zweiten Aspekt der Erfindung dazu ausgebildet sein, den Gasdruck in dem jeweiligen Zwischenraum bzw. die Leistung des mindestens einen Gasverdichters derart zu regeln, dass der Gasdruck und/oder die Leistung einen vorgegebenen Maximalwert nicht überschreiten und/oder einen vorgegebenen Minimalwert nicht unterschreiten, also in einem vorgegebenen Intervall bleiben. Es kann auch vorgesehen sein, den Gasdruck bzw. die Leistung des mindestens einen Gasverdichters auf einen bestimmten vorgegebenen Sollwert zu regeln. Die Maximal- und Minimalwerte bzw. Sollwerte können individuell für jeden Gasspeicher vorgegeben werden. Insbesondere nach dem ersten Aspekt kann als Gas beispielsweise Luft zur Druckerzeugung in den Zwischenraum zugeführt werden. Die Gaszuführeinrichtungen können in an sich bekannter Weise Stützluftgebläse sein. Für den Gastransport insbesondere nach dem zweiten Aspekt können an sich bekannte Gasverdichter zum Einsatz kommen.

In den Gasspeichern kann sich jeweils eine Gärsubstanz befinden. Durch die Erfindung wird in einfacher Weise eine genaue Einstellung des Gasdrucks in dem jeweiligen Zwischenraum bzw. der Leistung des mindestens einen Gasverdichters und damit auch des Gasdrucks über der in dem jeweiligen Gasspeicher gegebenenfalls enthaltenen Gärsubstanz ermöglicht. Dabei kann eine automatische Regelung erfolgen. Im Gegensatz zum Stand der Technik ist insbesondere bei dem ersten Aspekt der Erfindung kein Dauerbetrieb der Gaszuführeinrichtung mehr erforderlich. Es werden Energieeinsparungen erreicht. Außerdem kann durch die Regelung des Gasdrucks in dem Zwischenraum und damit auch in dem eigentlichen Gasspeicherraum gerade bei hohen Drucksituationen durch Abschaltung des Gebläses eine Entlastung der gegebenenfalls vorgesehenen Tragluftfolienabdeckung erreicht werden. Das System stabilisiert sich selbständig über die Regelfunktion. Da erfindungsgemäß der Druck in den Gasspeichern bzw. die Leistung der Gasverdichter mit einer höheren Genauigkeit geregelt werden können, sind auch geringe Differenzdrücke zwischen kaskadenartig in Serie geschalteten Gasspeichern realisierbar bzw. kann ein Gastransport über eine Druckerhöhung zwischen den Behältern sichergestellt werden. Dadurch lässt sich im Gegensatz zum Stand der Technik eine größere Zahl Gasspeicher verbinden. Dadurch wiederum lässt sich die Gasqualität steigern. Auch bei Wartungsvorgängen, bei denen beispielsweise ein schnelles Entleeren bzw. Umschichten der Gasspeicher erfolgen soll, bieten sich durch die Erfindung Vorteile. Insbesondere kann das Umschichten bzw. Entleeren durch gezielte Einflussnahme auf die Gasspeicherdrücke bzw. auf die Leistung der Gasverdichter beschleunigt werden. Zusammenfassend ist nach beiden Aspekten der Erfindung in einfacher und flexibler Weise ein effektives Gasspeicher- und Lastmanagement der Biogasanlage möglich.

Die erfindungsgemäße Vorrichtung und das erfindungsgemäße Verfahren können selbstverständlich zur Regelung der Gaszufuhr in Zwischenräume zwischen Gasspeichermembranen und Schutzabdeckungen bzw. der Leistung von Gasverdichtern von mehr als zwei Gasspeichern einer Biogasanlage dienen. Insbesondere kann die Biogasanlage dann beispielsweise 3, 4 oder mehr als 4 Gasspeicher aufweisen. Mit der Regeleinrichtung können dann wiederum in erfindungsgemäßer Weise die Zwischenraumdrücke sowie gegebenenfalls die Gärdrücke bzw. die Gasverdichterleistungen individuell für die einzelnen Gasspeicher regelbar sein. Die Gaszuführeinrichtungen der einzelnen Gasspeicher können jeweils eigene Stützluftgebläse aufweisen. Es ist aber auch denkbar, dass einige Bauteile der Gaszuführeinrichtungen gemeinsam für mehrere Gaszuführeinrichtungen vorgesehen sind. Im einfachsten Fall weisen die Gaszuführeinrichtungen jeweils nur eine individuelle Gaszuführleitung in den jeweiligen Zwischenraum auf. Es ist auch eine Kombination des ersten und zweiten Aspekts der Erfindung möglich.

Gemäß einer Ausgestaltung kann die Regeleinrichtung dazu ausgebildet sein, den Gasdruck in einem jeweiligen Zwischenraum eines Gasspeichers auf Grundlage eines voreingestellten Sollwerts für den Gasdruck in dem Zwischenraum dieses Gasspeichers sowie eines Sollwerts für den Gasdruck in dem Zwischenraum mindestens eines anderen Gasspeichers zu regeln. Bei dieser Ausgestaltung können für die Druckregelung der jeweiligen Gasspeicher also Messwerte der dem jeweiligen Gasspeicher zugeordneten Zwischenraumdruckmesseinrichtung und Messwerte von anderen Gasspeichern zugeordneten Zwischenraumdruckmesseinrichtungen, insbesondere sämtlichen Zwischenraumdruckmesseinrichtungen, berücksichtigt werden. Auf diese Weise ist bei einer Kaskadenanordnung mehrerer Gasspeicher, insbesondere mehrerer Gärbehälter, eine Abstimmung der Drücke in den einzelnen Gasspeichern aufeinander möglich. So kann beispielsweise eine Druckdifferenz zwischen in dem Gasverbund hintereinander angeordneten Gasspeichern vorgegeben und mittels der Regelung eingestellt werden. Der Gastransport zwischen den Gasspeichern kann so optimiert werden.

Gemäß einer weiteren Ausgestaltung kann vorgesehen sein, dass den Gasspeichern jeweils Speicherraumdruckmesseinrichtungen zugeordnet sind, mit denen der Gasdruck in den Speicherräumen gemessen werden kann und, dass die mindestens eine Regeleinrichtung dazu ausgebildet ist, die Leistung des mindestens einen Gasverdichters auf Grundlage der Messwerte der Speicherraumdruckmesseinrichtungen zumindest derjenigen Gasspeicher zu regeln, die durch die mit dem mindestens einen Gasverdichter versehene Gasüberströmleitung verbunden sind. Bei dieser Ausgestaltung kann der mindestens eine Gasverdichter über den Gasdruck der Behälter saug- als auch druckseitig der Gasverdichter geregelt werden. Bei den Speicherraumdruckmesseinrichtungen können Gasdrucksensoren zum Einsatz kommen, die kontinuierlich den anstehenden Gasdruck ausgeben, so dass über eine Auswerteeinheit die Gasverdichter geregelt werden können. Es kann sich aber beispielsweise auch um einfache Drückwächter handeln, die bei Über- bzw. Unterschreiten eines eingestellten Schwellwerts einen Schaltausgang schalten über den, mit oder ohne weiterreichender Regelung, der bzw. die Gasverdichter geregelt werden.

Nach einer weiteren Ausgestaltung können den Gasspeichern jeweils Füllstandsmesseinrichtungen zugeordnet sein, mit denen ein Gasfüllstand in den Speicherräumen der Gasspeicher gemessen werden kann, wobei die mindestens eine Regeleinrichtung dazu ausgebildet ist, die Leistung des mindestens einen Gasverdichters auf Grundlage der Messwerte der Füllstandsmesseinrichtungen zumindest derjenigen Gasspeicher zu regeln, die durch die mit dem mindestens einen Gasverdichter versehene Gasüberströmleitung verbunden sind. Der mindestens eine Gasverdichter wird also über den Füllstand der Gasspeicher saug- als auch druckseitig des Gasverdichters geregelt.

Der mindestens eine Gasverdichter kann weiterhin so dimensioniert sein, dass auch bei maximaler Leistung des Gasverdichters das Auftreten eines unzulässigen Über- oder Unterdrucks in einem der Gasspeicher vermieden wird. Der mindestens eine Gasverdichter ist also durch "Unterdimensionierung" nicht in der Lage, Schaden anzurichten bzw. kann gegebenenfalls durch Unter- und Überdruckschaltkontakte eine Notabschaltung erfahren.

Es kann weiterhin jedem Gasspeicher eine Regeleinrichtung zugeordnet sein. Eine Regelung der einzelnen Dachdrücke bzw. der einzelnen Gasverdichter kann also über voneinander unabhängige Regeleinheiten erfolgen.

Nach einer weiteren Ausgestaltung kann die Vorrichtung weiterhin Speicherraumdruckmesseinrichtungen umfassen, wobei jedem Gasspeicher mindestens eine Speicherraumdruckmesseinrichtung zugeordnet ist, mit der der Gasdruck in dem Gasspeicherraum unterhalb der Gasspeichermembran gemessen werden kann, und wobei die Messwerte der Speicherdruckmesseinrichtungen ebenfalls an der Regeleinrichtung anliegen und bei der Regelung des Gasdrucks in den Zwischenräumen berücksichtigt werden. Durch eine zusätzliche Messung des Gasdrucks in dem Speicherraum werden mehr Informationen über den Gesamtzustand der Anlage gewonnen, insbesondere inwiefern sich die Drücke in dem Zwischenraum und dem Speicherraum beispielsweise bei einer Dehnung der Gasspeicherfolie unterscheiden. Indem die zusätzliche Druckmessung bei der Regelung berücksichtigt wird, kann das System noch effektiver eingestellt werden. Dabei kann in den Gasspeichern eine Gärsubstanz enthalten sein. In diesem Fall wird von den Speicherraumdruckmesseinrichtungen der Gärdruck in dem Speicherraum über der Gärsubstanz gemessen. Der Gärdruck in dem Gasspeicher bezeichnet in diesem Zusammenhang den Druck in dem Speicherraum der Gasspeicher (direkt) über der jeweiligen Gärsubstanz. Wiederum können bei der Regelung des Drucks in einem Gasspeicher Messwerte der dem jeweiligen Gasspeicher zugeordneten Speicherraumdruckmesseinrichtung und Messwerte von anderen Gasspeichern zugeordneten Speicherraumdruckmesseinrichtungen, insbesondere sämtlichen Speicherraumdruckmesseinrichtungen, berücksichtigt werden.

Die Regeleinrichtung kann weiter dazu ausgebildet sein, die Gaszuführeinrichtungen so anzusteuern, dass der Gasdruck in den Zwischenräumen einen vorgegebenen Maximalwert nicht überschreiten. Die Regelung erfolgt somit auf Grundlage des jeweils höheren gemessenen Druckwerts. So wird verhindert, dass sich in den Zwischenräumen ein unzulässig hoher Druck ausbilden kann. Da sich die Drücke in dem Zwischenraum und dem Speicherraum, wie eingangs erläutert, gegenseitig beeinflussen, kann durch die Regelung der Gaszufuhr in den Zwischenraum auch der Druck in dem Speicherraum beeinflusst werden.

Wenn der Speicherraum unterhalb der Gasspeichermembran jedoch vollständig gefüllt ist, spannt sich die Gasspeichermembran und der Druck in dem Speicherraum steigt über den der Regeleinrichtung vorgegebenen Maximalwert an, so dass die Gaszuführeinrichtung ausgeschaltet bleibt. Um zu vermeiden, dass der Druck in dem Zwischenraum dadurch so weit absinkt, dass die Schutzabdeckung, beispielsweise die Wetterschutzfolie, ihre Formstabilität verliert, kann eine Mindestdruckregelung vorgesehen sein. Diese sieht vor, dass die Gaszuführeinrichtungen von der Regeleinrichtung so angesteuert werden, dass der Gasdruck in den Zwischenräumen der Gasspeicher einen vorgegebenen Minimalwert nicht unterschreitet.

Nach einer weiteren diesbezüglichen Ausgestaltung kann die Regeleinrichtung dazu ausgebildet sein, den Gasdruck in den Zwischenräumen so innerhalb des durch den Maximalwert und den Minimalwert vorgegebenen Intervalls zu regeln, dass der Gasdruck in dem Gasspeicherraum einem Sollwert möglichst nahe kommt. Bei dieser Ausgestaltung wird für den Druck in dem Speicherraum unterhalb der Gasspeichermembran, beispielsweise über einer Gärsubstanz, ein Sollwert vorgegeben, der ein optimiertes Gasspeicher- und Lastmanagement erlaubt, insbesondere einen optimierten Gastransport zwischen den Gasspeichern. Je nach den vorherrschenden Betriebs- und Druckverhältnissen ist es möglich, durch eine Regelung des Zwischenraumdrucks innerhalb des für den Zwischenraumdruck vorgegebenen zulässigen Intervalls einen Druck derart auszuwählen, dass der Speicherdruck in dem Gasspeicherraum dem vorgegebenen Sollwert möglichst nahe kommt bzw. diesen erreicht. Die Grenzen für diese indirekte Druckregelung in dem Speicherraum stellen die zulässigen Intervallgrenzen für den Zwischenraumdruck dar.

Die Maximal-, Minimal- und/oder Sollwerte für die Drücke können erfindungsgemäß für jeden Gasspeicher jeweils individuell vorgegeben werden.

Nach einer weiteren Ausgestaltung kann eine Zweipunktregelung durchgeführt werden. Die Gaszuführeinrichtung kann also insbesondere lediglich durch Einschalten oder Ausschalten angesteuert werden. Es ergibt sich dadurch eine besonders einfache Regelung, indem bei Unterschreiten eines vorgegebenen Minimalwerts für den Druck die Gaszuführung eingeschaltet wird, und bei Überschreiten eines vorgegebenen Maximalwerts für den Druck die Gaszuführung ausgeschaltet wird. Die bei Biogasanlagen vorherrschenden Systembedingungen lassen sich durch eine solche Zweipunktregelung oftmals ausreichend präzise abbilden. Selbstverständlich ist aber auch eine andere Regelung möglich, beispielsweise eine kontinuierliche Regelung über eine geeignete Regeleinrichtung, die beispielsweise Frequenzumrichter umfasst.

Die Zwischenräume der Gasspeicher können gemäß einer weiteren Ausgestaltung jeweils über mindestens eine druckregelnde Entlüftungsöffnung entlüftet werden. Eine solche Entlüftungsöffnung kann beispielsweise eine gewichtsbeaufschlagte Entlüftungsöffnung sein, beispielsweise eine Entlüftungsklappe, insbesondere eine Querstromklappe. Durch geeignete Gewichtsbeaufschlagung der Öffnung werden das Be- und Entlüftungsverhalten der Anlage und damit die Schaltfrequenzen der Regelung vorgegeben. Durch intelligentes Abstimmen der Druckregelung, beispielsweise einer Gewichtsbeaufschlagung, ist es möglich, für jeden Gasspeicher einen geeigneten Arbeitsbereich festzulegen, der durch die Regelung abgedeckt werden kann. Somit ist auch bei nach der Inbetriebnahme eintretenden Änderungen der Systembedingungen eine flexible Feinabstimmung über die Regelung möglich, ohne dass eine Änderung der Gewichtsbeaufschlagung erforderlich ist.

Nach einer weiteren vorteilhaften Ausgestaltung kann die Regeleinrichtung von einem von der Biogasanlage entfernten Ort steuerbar sein. Es ist also eine Fernbedienung der Regelung und damit eine Fernzugriffsmöglichkeit auf die Regelparameter in dem jeweils vorgegebenen Arbeitsbereich möglich. Ebenso lassen sich dann an einem von der Anlage entfernten Ort die jeweils herrschenden Bedingungen der Anlage überwachen.

Erfindungsgemäß kann das Gas in der Biogasanlage über einen längeren Zeitraum kontrolliert von einem Gasspeicher in den anderen geleitet werden. Insgesamt wird eine verbesserte Gasqualität erreicht. Das erfindungsgemäß ermöglichte Gasspeichermanagement erfolgt in flexibler, einfacher und präziser Weise. Ein Fernzugriff erlaubt eine komfortable Überwachung und Regelung des Systems.

Die Erfindung löst die Aufgabe auch durch eine Biogasanlage mit einer erfindungsgemäßen Vorrichtung. Die erfindungsgemäße Vorrichtung kann zur Durchführung des erfindungsgemäßen Verfahrens geeignet sein.

Ein Ausführungsbeispiel der Erfindung wird nachfolgend anhand einer Zeichnung näher erläutert. Es zeigen schematisch:
- Fig. 1: eine erfindungsgemäße Vorrichtung in einer teilgeschnittenen Ansicht,
- Fig. 2: ein Diagramm zur Veranschaulichung des Ansprechverhaltens bei verschiedenen Gewichtsbeaufschlagungen einer Entlüftungsklappe, und
- Fig. 3: ein weiteres Diagramm zur Veranschaulichung des Ansprechverhaltens in Abhängigkeit von der Gewichtsbeaufschlagung.

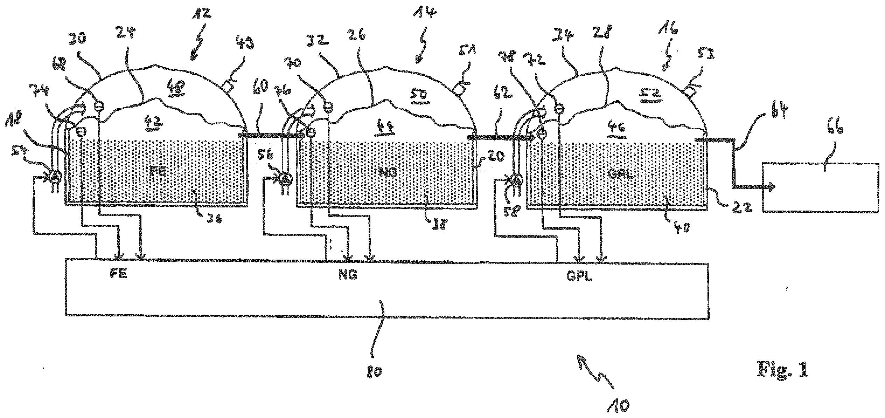

Soweit nichts anderes angegeben ist, bezeichnen in den Figuren gleiche Bezugszeichen gleiche Gegenstände. In Fig. 1 ist eine erfindungsgemäße Vorrichtung 10 zur Regelung der Gaszufuhr in einen Zwischenraum zwischen einer Gasspeichermembran und einer Schutzabdeckung von mindestens zwei Gasspeichern, vorliegend dreier Gärbehälter einer Biogasanlage, dargestellt. Die Biogasanlage weist drei Gasspeicher 12, 14, 16, vorliegend in Form dreier Behälter 12, 14, 16, auf, die kaskadenartig in Reihe geschaltet sind. Jeder der Behälter 12, 14, 16 ist zylindrisch ausgebildet. An der Kopfseite der jeweiligen Behälterwand 18, 20, 22 ist eine Gasspeichermembran, vorliegend eine Gasspeicherfolie 24, 26, 28, gasdicht und kraftschlüssig angebracht. Ebenfalls gasdicht und kraftschlüssig an dem Wandkopf und über der Gasspeicherfolie 24, 26, 28 ist eine Schutzabdeckung, vorliegend eine Wetterschutzfolie 30, 32, 34, befestigt. Die Folien bilden als Doppelmembran eine Tragluftfolienabdeckung. In die Behälter 12, 14, 16 ist eine Gärsubstanz 36, 38, 40 zum Erzeugen von Biogas gefüllt. Das über der Gärsubstanz 36, 38, 40 entstehende Biogas füllt die jeweiligen Speicherräume 42, 44, 46 über der jeweiligen Gärsubstanz 36, 38, 40 und unterhalb der Gasspeicherfolien 24, 26, 28. In die jeweiligen Zwischenräume 48, 50, 52 zwischen den Gasspeicherfolien 24, 26, 28 und den Wetterschutzfolien 30, 32, 34 wird jeweils Gas, im dargestellten Beispiel Luft, eingeblasen. Dazu ist jedem Behälter 12, 14, 16 eine Gaszuführeinrichtung 54, 56, 58, vorliegend jeweils ein Stützluftgebläse 54, 56, 58, zugeordnet. Darüber hinaus ist jedem Zwischenraum 48, 50, 52 mindestens eine Entlüftungsklappe 49, 51, 53, in dem gezeigten Beispiel jeweils eine Querstromklappe 49, 51, 53, zugeordnet. Die Entlüftungsklappen 49, 51, 53 werden mit Gewichten beaufschlagt, die den Staudruck des Luftauslasses regulieren. Die Wetterschutzfolien 30, 32, 34 werden auf diese Weise formstabil gehalten. Die Gasspeicherfolien 24, 26, 28 können sich dagegen je nach Füllstand in den Speicherräumen 42, 44, 46 auf und ab bewegen. Die einzelnen Behälter 12, 14, 16 sind über Gasüberströmleitungen 60, 62 miteinander verbunden. In dem Speicherraum 42 des ersten Behälters 12 entstehendes Gas kann über die Leitung 60 in den Speicherraum 44 des zweiten Behälters 14 gelangen. Gas aus dem Speicherraum 44 des zweiten Behälters 14 wiederum kann über die Leitung 62 in den Speicherraum 46 des dritten Behälters 16 gelangen. Der erste Behälter ist ein sogenannter Fermenter (FE), der zweite Behälter ein sogenannter Nachgärer (NG) und der dritte Behälter ein so genanntes Gärproduktlager (GPL). Aus dem dritten Behälter 16 gelangt das Gas über eine Leitung 64 zur weiteren Verwertung, beispielsweise einem Verbraucher 66. Dieser Aufbau einer Biogasanlage ist an sich bekannt.

In jedem Zwischenraum 48, 50, 52 ist weiterhin jeweils eine Zwischenraumdruckmesseinrichtung 68, 70, 72 angeordnet, die den Gasdruck in dem jeweiligen Zwischenraum 48, 50, 52 misst. Darüber hinaus ist in jedem der Speicherräume 42, 44, 46 eine Speicherraumdruckmesseinrichtung 74, 76, 78, in dem dargestellten Beispiel eine Gärdruckmesseinrichtung 74, 76, 78, angeordnet, die den Gasdruck in dem jeweiligen Speicherraum 42, 44, 46 unterhalb den Gasspeicherfolien 24, 26, 28 und über der jeweiligen Gärsubstanz 36, 38, 40 misst. Die Messwerte der Druckmesseinrichtungen 68, 70, 72 und 74, 76, 78 liegen an einer Regeleinrichtung 80 an. Die Regeleinrichtung 80 regelt den Gasdruck in den Zwischenräumen 48, 50, 52 der Behälter 12, 14, 16 sowie in den Speicherräumen 42, 44, 46 individuell auf Grundlage der jeweiligen Messwerte der Messeinrichtungen 68, 70, 72 und 74, 76, 78. Dazu steuert die Regeleinrichtung 80 die den Behältern 12, 14, 16 jeweils zugeordneten Gaszuführeinrichtungen 54, 56, 58 in geeigneter Weise individuell an. In dem dargestellten Beispiel steuert die Regeleinrichtung 80 die Gebläse 54, 56, 58 jeweils so an, dass in jedem Behälter 12, 14, 16 sowohl der Zwischenraumdruck als auch der Speicherraumdruck unterhalb eines vorgegebenen Maximalwerts bleiben. Darüber hinaus steuert die Regeleinrichtung 80 die Gebläse 54, 56, 58 so an, dass der Zwischenraumdruck einen Mindestwert nicht unterschreitet, so dass die Formstabilität der Wetterschutzfolie zu jeder Zeit gewährleistet ist. In dem dargestellten Beispiel ist die Regeleinrichtung 80 an dem Ort der Biogasanlage angeordnet und beispielsweise über eine geeignete Fernbedienungseinrichtung von einem von der Anlage entfernten Ort steuerbar.

Alternativ oder zusätzlich zu der oben beschriebenen Zwischenraumdruckregelung könnte auch ein Gastransport über eine Leistungsregelung von Gasverdichtern erfolgen. Dazu kann in den Gasüberströmleitungen 60, 62 zwischen den Gasspeichern 12, 14, 16 jeweils ein nicht dargestellter Gasverdichter angeordnet sein. Die Leistung der Gasverdichter kann von der Regeleinrichtung 80 dann jeweils beispielsweise auf Grundlage des gemessenen Gasdrucks in den Gasspeicherräumen jeweils derjenigen Gasspeicher geregelt werden, die durch die mit dem jeweiligen Gasverdichter versehene Gasüberströmleitung 60, 62 verbunden sind. Alternativ oder zusätzlich kann eine Regelung der Gasverdichter auch jeweils auf Grundlage des gemessenen Füllstands zumindest derjenigen Gasspeicher erfolgen, die durch die mit dem jeweiligen Gasverdichter versehene Gasüberströmleitung (60, 62) verbunden sind.

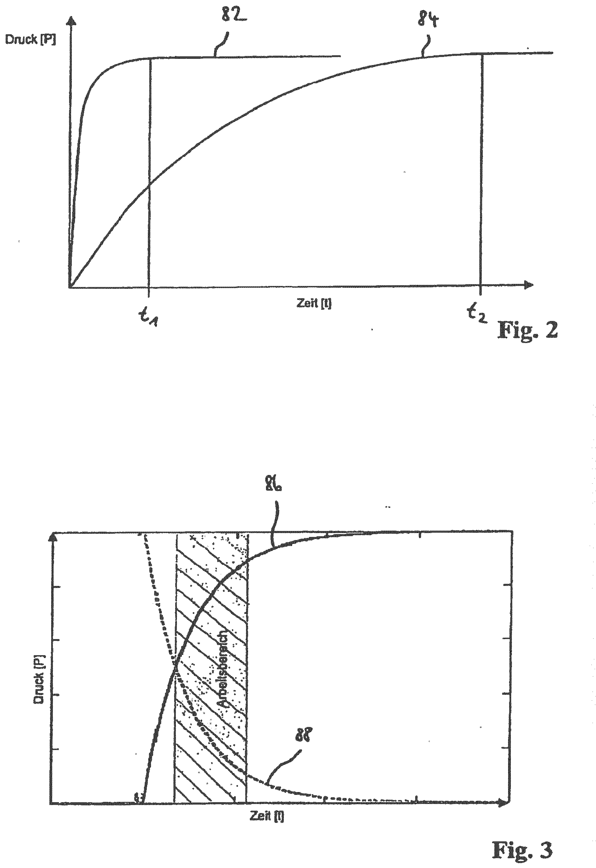

In Fig. 2 ist ein Diagramm zur Veranschaulichung der Auswirkungen unterschiedlicher Gewichtsbeaufschlagungen auf die Entlüftungsklappen 49, 51, 53 der Zwischenräume 48, 50, 52 der Behälter 12, 14, 16 der Biogasanlage gezeigt. Dabei ist der Druck P in dem jeweiligen Zwischenraum 48, 50, 52 über der Zeit t beim Belüften der Zwischenräume 48, 50, 52 aufgetragen. Die Kurve 82 zeigt exemplarisch einen Druckanstiegsverlauf bei konstanter Leistung der Stützluftgebläse 54, 56, 58 und einer hohen Gewichtsbeaufschlagung der Entlüftungsklappen 49, 51, 53. Der Druck P steigt in kurzer Zeit steil an und erreicht zu einem Zeitpunkt t₁ eine Sättigung. Die Kurve 84 in Fig. 2 zeigt den Druckverlauf bei derselben Leistung der Stützluftgebläse 54, 56, 58 wie bei der Kurve 82, jedoch bei einer geringen Gewichtsbeaufschlagung der Entlüftungsklappen 49, 51, 53. Der Druck P in dem Zwischenraum steigt erheblich langsamer an und erreicht die Sättigung erst zu einem erheblich späteren Zeitpunkt t₂.

Fig. 3 zeigt ein weiteres Diagramm zur Veranschaulichung des Ansprechverhaltens beim Be- und Entlüften der Biogasanlage bei konstanter Leistung der Stützluftgebläse 54, 56, 58. Wiederum ist der Druck P in dem jeweiligen Zwischenraum 48, 50, 52 über der Zeit t aufgetragen. Die Kurve 86 zeigt den Druckverlauf beim Belüften der jeweiligen Zwischenräume 48, 50, 52. Die Kurve 88 in Fig. 3 zeigt entsprechend den Druckverlauf beim Entlüften der Zwischenräume 48, 50, 52. Der in dem Diagramm schraffierte Bereich stellt den Arbeitsbereich dar, innerhalb dessen die Drücke in den Zwischenräumen 48, 50, 52 mittels der Regeleinrichtung im Betrieb geregelt werden können.

Im Betrieb der Anlage muss ein Kompromiss bei der Gewichtsbeaufschlagung gefunden werden, der es erlaubt, mittels der Regeleinrichtung 80 möglichst sämtliche im Rahmen des Betriebs der Anlage erforderlichen Drücke in den Zwischenräumen 48, 50, 52 bzw. den Speicherräumen 42, 44, 46 einzustellen.

## Patentansprüche

1. Vorrichtung zur Regelung der Gaszufuhr bei einem Gasspeichersystem einer Biogasanlage, wobei das Gasspeichersystem mindestens zwei kaskadenartig in Reihe geschaltete Gasspeicher aufweist, die jeweils einen Zwischenraum zwischen einer Gasspeichermembran und einer Schutzabdeckung besitzen
- die Vorrichtung umfasst Gaszuführeinrichtungen (54, 56, 58), Zwischenraumdruckmesseinrichtungen (68, 70, 72) und mindestens eine Regeleinrichtung (80),
- wobei jedem Gasspeicher (12, 14, 16) jeweils mindestens eine Gaszuführeinrichtung (54, 56, 58) zugeordnet ist, mit der dem Zwischenraum (48, 50, 52) des jeweiligen Gasspeichers (12, 14, 16) Gas zugeführt werden kann,
- wobei jedem Gasspeicher (12, 14, 16) jeweils mindestens eine Zwischenraumdruckmesseinrichtung (68, 70, 72) zugeordnet ist, mit der der Gasdruck in dem Zwischenraum (48, 50, 52) des jeweiligen Gasspeichers (12, 14, 16) gemessen werden kann,
- wobei die Messwerte der Zwischenraumdruckmesseinrichtungen (68, 70, 72) an der mindestens einen Regeleinrichtung (80) anliegen, und
- wobei die mindestens eine Regeleinrichtung (80) dazu ausgebildet ist, auf Grundlage der Messwerte der Zwischenraumdruckmesseinrichtungen (68, 70, 72) den Gasdruck in den Zwischenräumen (48, 50, 52) der Gasspeicher (12, 14, 16) durch eine Ansteuerung der den Gasspeichern (12, 14, 16) jeweils zugeordneten Gaszuführeinrichtungen (54, 56, 58) zu regeln.

2. Vorrichtung nach Anspruch 1, **dadurch gekennzeichnet, dass** die mindestens eine Regeleinrichtung dazu ausgebildet ist, den Gasdruck in einem jeweiligen Zwischenraum (48, 50, 52) eines Gasspeichers (12, 14, 16) auf Grundlage eines vorgegebenen Sollwerts für den Gasdruck in dem Zwischenraum dieses Gasspeichers sowie eines Sollwerts für den Gasdruck in dem Zwischenraum mindestens eines anderen Gasspeichers zu regeln.

3. Vorrichtung zur Regelung des Gastransports bei einem Gasspeichersystem einer Biogasanlage, wobei das Gasspeichersystem mindestens zwei kaskadenartig in Reihe geschaltete Gasspeicher aufweist, die jeweils einen Zwischenraum zwischen einer Gasspeichermembran und einer Schutzabdeckung besitzen,
- wobei die Vorrichtung mindestens eine Regeleinrichtung (80) umfasst,
- wobei jeweils Speicherräume (42, 44, 46) der Gasspeicher (12, 14, 16) für Gas durch mindestens eine Gasüberströmleitung (60, 62) miteinander verbunden sind,
- wobei in der mindestens einen Gasüberströmleitung (60, 62) mindestens ein Gasverdichter angeordnet ist, und
- wobei die mindestens eine Regeleinrichtung (80) dazu ausgebildet ist, die Leistung des mindestens einen Gasverdichters so zu regeln, dass über die mindestens eine Gasüberströmleitung (60, 62) ein Gastransport zwischen den Speicherräumen (42, 44, 46) der Gasspeicher erfolgt.

4. Vorrichtung nach Anspruch 3, **dadurch gekennzeichnet, dass** den Gasspeichern (12, 14, 16) jeweils Speicherraumdruckmesseinrichtungen (74, 76, 78) zugeordnet sind, mit denen der Gasdruck in den Speicherräumen (42, 44, 46) gemessen werden kann und, dass die mindestens eine Regeleinrichtung (80) dazu ausgebildet ist, die Leistung des mindestens einen Gasverdichters auf Grundlage der Messwerte der Speicherraumdruckmesseinrichtungen (74, 76, 78) zumindest derjenigen Gasspeicher (12, 14, 16) zu regeln, die durch die mit dem mindestens einen Gasverdichter versehene Gasüberströmleitung (60, 62) verbunden sind, und/oder dass den Gasspeichern (12, 14, 16) jeweils Füllstandsmesseinrichtungen zugeordnet sind, mit denen ein Gasfüllstand in den Speicherräumen (42, 44, 46) der Gasspeicher (12, 14, 16) gemessen werden kann und, dass die mindestens eine Regeleinrichtung (80) dazu ausgebildet ist, die Leistung des mindestens einen Gasverdichters auf Grundlage der Messwerte der Füllstandsmesseinrichtungen zumindest derjenigen Gasspeicher (12, 14, 16) zu regeln, die durch die mit dem mindestens einen Gasverdichter versehene Gasüberströmleitung (60, 62) verbunden sind.

5. Vorrichtung nach einem der Ansprüche 3 oder 4, **dadurch gekennzeichnet, dass** der mindestens eine Gasverdichter so dimensioniert ist, dass auch bei maximaler Leistung des Gasverdichters das Auftreten eines unzulässigen Über- oder Unterdrucks in einem der Gasspeicher vermieden wird.

6. Vorrichtung nach einem der vorhergehenden Ansprüche, **dadurch gekennzeichnet, dass** die Vorrichtung weiterhin Speicherraumdruckmesseinrichtungen (74, 76, 78) umfasst, wobei jedem Gasspeicher (12, 14, 16) jeweils mindestens eine Speicherraumdruckmesseinrichtung (74, 76, 78) zugeordnet ist, mit der der Gasdruck in dem Gasspeicherraum unterhalb der Gasspeichermembran gemessen werden kann, und wobei die Messwerte der Speicherraumdruckmesseinrichtungen (74, 76, 78) ebenfalls an der Regeleinrichtung (80) anliegen und bei der Regelung des Gasdrucks in den Zwischenräumen (48, 50, 52) berücksichtigt werden.

7. Vorrichtung nach einem der vorhergehenden Ansprüche, **dadurch gekennzeichnet, dass** die Regeleinrichtung (80) dazu ausgebildet ist, die Gaszuführeinrichtungen (54, 56, 58) so anzusteuern, dass der Gasdruck in den Zwischenräumen (48, 50, 52) einen vorgegebenen Maximalwert nicht überschreiten, und/oder dass die Regeleinrichtung (80) dazu ausgebildet ist, die Gaszuführeinrichtungen (54, 56, 58) so anzusteuern, dass der Gasdruck in den Zwischenräumen (48, 50, 52) einen vorgegebenen Minimalwert nicht unterschreitet.

8. Vorrichtung nach Anspruch 7, **dadurch gekennzeichnet, dass** die Regeleinrichtung (80) dazu ausgebildet ist, den Gasdruck in den Zwischenräumen (48, 50, 52) so innerhalb des durch den Maximalwert und den Minimalwert vorgegebenen Intervalls zu regeln, dass der Gasdruck in dem Gasspeicherraum einem Sollwert möglichst nahe kommt.

9. Vorrichtung nach einem der vorhergehenden Ansprüche, **dadurch gekennzeichnet, dass** die Zwischenräume (48, 50, 52) jeweils mindestens eine druckregelnde Entlüftungsöffnung (49, 51, 53) aufweisen.

10. Verfahren zur Regelung der Gaszufuhr bei einem Gasspeichersystem einer Biogasanlage, wobei das Gasspeichersystem mindestens zwei kaskadenartig in Reihe geschaltete Gasspeicher aufweist, die jeweils einen Zwischenraum zwischen einer Gasspeichermembran und einer Schutzabdeckung besitzen, umfassend die Schritte:
- den Zwischenräumen (48, 50, 52) der Gasspeicher (12, 14, 16) wird jeweils Gas zugeführt,
- der Gasdruck in den Zwischenräumen (48, 50, 52) wird jeweils gemessen, und
- auf Grundlage des gemessenen Gasdrucks in den Zwischenräumen (48, 50, 52) wird jeweils durch eine Ansteuerung der Gaszufuhr in die Zwischenräume (48, 50, 52) der Gasdruck in den Zwischenräumen (48, 50, 52) geregelt.

11. Verfahren nach Anspruch 10, **dadurch gekennzeichnet, dass** der Gasdruck in einem jeweiligen Zwischenraum (48, 50, 52) eines Gasspeichers auf Grundlage eines Sollwerts für den Gasdruck in dem Zwischenraum dieses Gasspeichers sowie eines Sollwerts für den Gasdruck in dem Zwischenraum mindestens eines anderen Gasspeichers geregelt wird.

12. Verfahren zur Regelung des Gastransports bei einem Gasspeichersystem einer Biogasanlage, wobei das Gasspeichersystem mindestens zwei kaskadenartig in Reihe geschaltete Gasspeicher aufweist, die jeweils einen Zwischenraum zwischen einer Gasspeichermembran und einer Schutzabdeckung besitzen,
- wobei jeweils Speicherräume (42, 44, 46) der Gasspeicher (12, 14, 16) für Gas durch mindestens eine Gasüberströmleitung (60, 62) miteinander verbunden sind,
- wobei in der mindestens einen Gasüberströmleitung (60, 62) mindestens ein Gasverdichter angeordnet ist, und
- wobei die Leistung des mindestens einen Gasverdichters so geregelt wird, dass über die mindestens eine Gasüberströmleitung (60, 62) ein Gastransport zwischen den Speicherräumen (42, 44, 46) der Gasspeicher erfolgt.

13. Verfahren nach Anspruch 12, **dadurch gekennzeichnet, dass** der Gasdruck in den Speicherräumen (42, 44, 46) gemessen wird und, dass die Leistung des mindestens einen Gasverdichters auf Grundlage des gemessenen Gasdrucks zumindest derjenigen Gasspeicher (12, 14, 16) geregelt wird, die durch die mit dem mindestens einen Gasverdichter versehene Gasüberströmleitung (60, 62) verbunden sind, und/oder dass der Gasfüllstand in den Speicherräumen (42, 44, 46) gemessen wird und, dass die Leistung des mindestens einen Gasverdichters auf Grundlage des gemessenen Gasfüllstands zumindest derjenigen Gasspeicher (12, 14, 16) geregelt wird, die durch die mit dem mindestens einen Gasverdichter versehene Gasüberströmleitung (60, 62) verbunden sind.

14. Verfahren nach einem der Ansprüche 12 oder 13, **dadurch gekennzeichnet, dass** der mindestens eine Gasverdichter so dimensioniert ist, dass auch bei maximaler Leistung des Gasverdichters das Auftreten eines unzulässigen Über- oder Unterdrucks in einem der Gasspeicher vermieden wird.

15. Verfahren nach einem der Ansprüche 10 bis 14, **dadurch gekennzeichnet, dass** weiterhin der Gasdruck (12, 14, 16) in dem Gasspeicherraum unterhalb der Gasspeichermembran gemessen wird, wobei der in dem Gasspeicherraum gemessene Gasdruck bei der Regelung des Gasdrucks in den Zwischenräumen (48, 50, 52) berücksichtigt werden.

16. Verfahren nach Anspruch 15, **dadurch gekennzeichnet, dass** die Gaszufuhr so angesteuert wird, dass der Gasdruck in den Zwischenräumen (48, 50, 52) einen vorgegebenen Maximalwert nicht überschreiten, und/oder dass die Gaszufuhr so angesteuert wird, dass der Gasdruck in den Zwischenräumen (48, 50, 52) einen vorgegebenen Minimalwert nicht unterschreitet.

17. Verfahren nach Anspruch 16, **dadurch gekennzeichnet, dass** der Gasdruck in den Zwischenräumen (48, 50, 52) so innerhalb des durch den Maximalwert und den Minimalwert vorgegebenen Intervalls geregelt wird, dass der Gasdruck in dem Gasspeicherraum einem Sollwert möglichst nahe kommt.

18. Verfahren nach einem der Ansprüche 10 bis 17, **dadurch gekennzeichnet, dass** die Zwischenräume (48, 50, 52) jeweils über mindestens eine druckregelnde Entlüftungsöffnung (49, 51, 53) entlüftet werden.

## Claims

1. A device for regulating the gas supply in a gas storage system of a biogas system, wherein the gas storage system has at least two gas stores connected in series in a cascade-like manner that each have an intermediate chamber between a gas store membrane and a protective covering
- the device comprises gas supply means (54, 56, 58), intermediate chamber pressure measuring means (68, 70, 72) and at least one regulating means (80),
- wherein to each gas store (12, 14, 16) at least one gas supply means (54, 56, 58) is assigned with which gas can be supplied to the intermediate chamber (48, 50, 52) of the respective gas store (12, 14, 16),
- wherein to each gas store (12, 14, 16) at least one intermediate chamber pressure measuring means (68, 70, 72) is assigned with which the gas pressure in the intermediate chamber (48, 50, 52) of the respective gas store (12, 14, 16) can be measured,
- wherein the measured values of the intermediate chamber pressure measuring means (68, 70, 72) are supplied to at least one regulating means (80), and
- wherein the at least one regulating means (80) is designed to regulate the gas pressure in the intermediate chambers (48, 50, 52) of the gas stores (12, 14, 16), on the basis of the measured values of the intermediate chamber pressure measuring means (68, 70, 72), by a control of the gas supply means (54, 56, 58) assigned respectively to the gas stores (12, 14, 16).

2. The device according to claim 1, **characterized in that** the at least one regulating means is designed to regulate the gas pressure in a respective intermediate chamber (48, 50, 52) of a gas store (12, 14, 16), on the basis of a specified set value for the gas pressure in the intermediate chamber of this gas store and a set value for the gas pressure in the intermediate chamber of at least one other gas store.

3. A device for regulating the gas transport in a gas storage system of a biogas system, wherein the gas storage system has at least two gas stores connected in series in a cascade-like manner that each have an intermediate chamber between a gas store membrane and a protective covering,
- wherein the device comprises at least one regulating means (80),
- wherein store chambers (42, 44, 46) of the gas stores (12, 14, 16) are connected together by at least one gas overflow line (60, 62),
- wherein at least one gas condenser is disposed in the at least one gas overflow line (60, 62), and
- wherein the at least one regulating means (80) is designed to regulate the performance of the at least one gas condenser so that a gas transport between the store chambers (42, 44, 46) of the gas stores occurs via the at least one gas overflow line (60, 62).

4. The device according to claim 3, **characterized in that** to the gas stores (12, 14, 16) store chamber pressure measuring means (74, 76, 78) are respectively assigned with which the gas pressure in the store chambers (42, 44, 46) can be measured, and that the at least one regulating means (80) is designed to regulate the power of the at least one gas condenser, on the basis of the measure values of the store chamber pressure measuring means (74, 76, 78) at least of those respective gas stores (12, 14, 16) that are connected by the gas overflow line (60, 62) provided with the at least gas condenser, and/or that the gas stores (12, 14, 16) are each assigned fill level measuring means with which a fill level in the store chambers (42, 44, 46) of the gas stores (12, 14, 16) can be measured, and that the at least one regulating means (80) is designed to regulate the power of the at least one gas condenser, on the basis of the measured values of the fill level measuring means at least of those respective gas stores (12, 14, 16), which are connected by the gas overflow line (60, 62) provided with the at least one gas condenser.

5. The device according to one of the claims 3 or 4, **characterized in that** the at least one gas condenser is dimensioned so that even at maximum power of the gas condenser the occurrence of an impermissible overpressure or underpressure in one of the gas stores is avoided.

6. The device according to one of the preceding claims, **characterized in that** the device further comprises store chamber measuring means (74, 76, 78), wherein each gas store (12, 14, 16) is assigned at least one store chamber measuring means (74, 76, 78), with which the gas pressure in the gas store chamber below the gas store membrane can be measured, and wherein the measure values of the store chamber measuring means (74, 76, 78) are also transmitted to the regulating means (80) and are considered with the regulation of the gas pressure in the intermediate chambers (48, 50, 52).

7. The device according to one of the preceding claims, **characterized in that** the regulating means (80) is designed to control the gas supply means (54, 56, 58) so that the gas pressure in the intermediate chambers (48, 50, 52) does not exceed a specified maximum value, and/or that the regulating means (80) is designed to control the gas supply means (54, 56, 58) so that the gas pressure in the intermediate chambers (48, 50, 52) does not fall below a specified minimum value.

8. The device according to claim 7, **characterized in that** the regulating means (80) is designed to regulate the gas pressure in the intermediate chambers (48, 50, 52) within the interval specified by the maximum value and the minimum value, so that the gas pressure in the gas store chamber comes as close as possible to a set value.

9. The device according to one of the preceding claims, **characterized in that** the intermediate chambers (48, 50, 52) each have at least one pressure regulating ventilation opening (49, 51, 53).

10. A method for regulating the gas supply in a gas storage system of a biogas system, wherein the gas storage system has at least two gas stores connected in series in a cascade-like manner, each of which has an intermediate chamber between a gas store membrane and a protective cover, comprising the steps:
- supplying gas to each of the intermediate chambers (48, 50, 52) of the gas stores (12, 14, 16),
- measuring the gas pressure in each of the intermediate chambers (48, 50, 52), and
- regulating the gas pressure in the intermediate chambers (48, 50, 52), on the basis of the measured gas pressures in the intermediate chambers (48, 50, 52), by controlling the gas supply into the intermediate chambers (48, 50, 52).

11. The method according to claim 10, **characterized in that** the gas pressure in a respective store chamber (48, 50, 52) of a gas store is regulated, on the basis of a set value for the gas pressure in the intermediate chamber of this gas store, and a set value for the gas pressure in the intermediate chamber of at least another gas store.

12. A method for regulating the gas transport in a gas storage system of a biogas system, wherein the gas storage system has at least two gas stores connected in series in a cascade-like manner that each have an intermediate chamber between a gas store membrane and a protective covering,
- wherein store chambers (42, 44, 46) of the gas stores (12, 14, 16) are connected together by at least one gas overflow line (60, 62),
- wherein at least one gas condenser is disposed in the at least one gas overflow line (60, 62), and
- wherein the power of the at least one gas condenser is regulated so that gas is transported between the store chambers (42, 44, 46) of the gas stores via the at least one overflow line (60, 62).

13. The method according to claim 12, **characterized in that** the gas pressure in the store chambers (42, 44, 46) is measured, and that the power of the at least one gas condenser is regulated, on the basis of the measured gas pressure in at least those gas stores (12, 14, 16), which are connected by the gas overflow line (60, 62) provided with the at least one gas condenser and/or that the gas fill level in the store chambers (42, 44, 46) is measured, and that the power of the at least one gas condenser is regulated, on the basis of the measured gas fill level of at least those gas stores (12, 14, 16), which are connected together by the gas overflow line (60, 62) provided with the at least one gas condenser.

14. The method according to one of the claims 12 or 13, **characterized in that** the at least one gas condenser is dimensioned so that even at maximum power of the gas condenser the occurrence of a impermissible overpressure or underpressure in one of the gas stores is avoided.

15. The method according to one of the claims 10 to 14, **characterized in that** further the gas pressure (12, 14, 16) in the gas store chamber below the gas store membrane is measured, wherein the gas pressure measured in the gas store chamber is considered during regulation of the gas pressure in the intermediate chambers (48, 50, 52).

16. The method according to claim 15, **characterized in that** the gas supply is controlled so that the gas pressure in the intermediate chambers (48, 50, 52) does not exceed a specified maximum value and/or that the gas supply is controlled so that the gas pressure in the intermediate chambers (48, 50, 52) does not fall below a specified minimum value.

17. The method according to claim 16, **characterized in that** the gas pressure in the intermediate chambers (48, 50, 52) is regulated within an interval specified by the maximum value and the minimum value so that the gas pressure in the gas store chambers comes as close as possible to a set value.

18. The method according to one of the claims 10 to 17, **characterized in that** the intermediate chambers (48, 50, 52) are each ventilated using at least one pressure regulating ventilation opening (49, 51, 53).

## Revendications

1. Dispositif de régulation d'apport de gaz dans un système de stockage de gaz d'une installation biogaz, dans lequel le système de stockage de gaz comprend au moins deux accumulateurs de gaz commutés en cascade en série qui possèdent respectivement un espace intermédiaire entre une membrane d'accumulateur de gaz et un revêtement de protection
- le dispositif comprend des installations d'apport de gaz (54, 56, 58), des installations de mesure de la pression de l'espace intermédiaire (68, 70, 72) et au moins une installation de régulation (80),
- chaque accumulateur de gaz (12, 14, 16) est équipé respectivement d'au moins une installation d'apport de gaz (54, 56, 58), qui permet d'amener du gaz à l'espace intermédiaire (48, 50, 52) de l'accumulateur de gaz respectif (12, 14, 16),
- chaque accumulateur de gaz (12, 14, 16) est équipé respectivement d'au moins une installation de mesure de la pression de l'espace intermédiaire (68, 70, 72), qui permet de mesurer la pression de gaz dans l'espace intermédiaire (48, 50, 52) de l'accumulateur de gaz respectif (12, 14, 16),
- les valeurs de mesure des installations de mesure de la pression de l'espace intermédiaire (68, 70, 72) sont ajustées sur ladite au moins une installation de régulation (80), et
- ladite au moins une installation de régulation (80) est conçue de manière à réguler sur la base des valeurs de mesure des installations de mesure de la pression de l'espace intermédiaire (68, 70, 72) la pression de gaz dans les espaces intermédiaires (48, 50, 52) des accumulateurs de gaz (12, 14, 16) par une commande des installations d'apport de gaz (54, 56, 58) respectivement attribuées aux accumulateurs de gaz (12, 14, 16).

2. Dispositif selon la revendication 1, **caractérisé en ce que** ladite au moins une installation de régulation est conçue de manière à réguler la pression de gaz dans un espace intermédiaire respectif (48, 50, 52) d'un accumulateur de gaz (12, 14, 16) sur la base d'une valeur de consigne définie pour la pression de gaz dans l'espace intermédiaire de cet accumulateur de gaz, ainsi que sur la base d'une valeur de consigne pour la pression de gaz dans l'espace intermédiaire d'au moins un autre accumulateur de gaz.

3. Dispositif de régulation du transport de gaz dans un système de stockage de gaz d'une installation biogaz, dans lequel le système de stockage de gaz comprend au moins deux accumulateurs de gaz commutés en cascade en série qui possèdent respectivement un espace intermédiaire entre une membrane d'accumulateur de gaz et un revêtement de protection
- le dispositif comprenant au moins une installation de régulation (80),
- des espaces de stockage (42, 44, 46) des accumulateurs de gaz (12, 14, 16) pour le gaz étant reliés respectivement les uns aux autres par au moins une conduite de trop-plein de gaz (60, 62),
- au moins un compresseur à gaz étant disposé dans ladite au moins une conduite de trop-plein de gaz (60, 62), et
- ladite au moins une installation de régulation (80) étant conçue pour réguler le rendement d'au moins un compresseur à gaz de telle sorte qu'un transport de gaz est assuré entre les espaces de stockage (42, 44, 46) via ladite au moins une conduite de trop-plein de gaz (60, 62).

4. Dispositif selon la revendication 3, **caractérisé en ce que** les accumulateurs de gaz (12, 14, 16) sont respectivement équipés d'installations de mesure de la pression de l'espace de stockage (74, 76, 78) qui permettent de mesurer la pression de gaz dans les espaces de stockage (42, 44, 46), et **en ce que** ladite au moins une installation de régulation (80) est conçue de manière à réguler le rendement dudit au moins un compresseur à gaz sur la base des valeurs de mesure des installations de mesure de la pression de l'espace de stockage (74, 76, 78) d'au moins ces accumulateurs de gaz (12, 14, 16) qui sont reliés par la conduite de trop-plein de gaz (60, 62) munie d'au moins un compresseur à gaz, et/ou **en ce que** les accumulateurs de gaz (12, 14, 16) sont respectivement équipés d'installations de mesure du niveau de remplissage qui permettent de mesurer un niveau de remplissage de gaz dans les espaces de stockage (42, 44, 46) des accumulateurs de gaz (12, 14, 16) et, **en ce que** ladite au moins une installation de régulation (80) est conçue de manière à réguler le rendement d'au moins un compresseur à gaz sur la base des valeurs de mesure des installations de mesure du niveau de remplissage d'au moins ces accumulateurs de gaz (12, 14, 16) qui sont reliés par la conduite de trop-plein de gaz (60, 62) munie d'au moins un compresseur à gaz.

5. Dispositif selon une des revendications 3 ou 4, **caractérisé en ce que** ledit au moins un compresseur à gaz est dimensionné de manière à éviter la survenance d'une surpression ou dépression non autorisée dans un des accumulateurs de gaz même en cas de rendement maximal du compresseur à gaz.

6. Dispositif selon une des revendications précédentes, **caractérisé en ce que** le dispositif comprend également des installations de mesure de la pression de l'espace de stockage (74, 76, 78), dans lequel chaque accumulateur de gaz (12, 14, 16) est muni d'au moins une installation de mesure de la pression de l'espace de stockage (74, 76, 78) qui permet de mesurer la pression de gaz dans l'espace de stockage de gaz en dessous de la membrane d'accumulateur de gaz, et dans lequel les valeurs de mesure des installations de mesure de la pression de l'espace de stockage (74, 76, 78) sont également ajustées à l'installation de régulation (80) et sont prises en compte lors de la régulation de la pression de gaz dans les espaces intermédiaires (48, 50, 52).

7. Dispositif selon une des revendications précédentes, **caractérisé en ce que** l'installation de régulation (80) est conçue de manière à commander les installations d'apport de gaz (54, 56, 58), de sorte que la pression de gaz dans les espaces intermédiaires (48, 50, 52) ne dépasse pas une valeur maximale définie, et/ou **en ce que** l'installation de régulation (80) est conçue de manière à commander les installations d'apport de gaz (54, 56, 58), de sorte que la pression de gaz dans les espaces intermédiaires (48, 50, 52) ne passe pas en dessous d'une valeur minimale définie.

8. Dispositif selon la revendication 7, **caractérisé en ce que** l'installation de régulation (80) est conçue de manière à réguler la pression de gaz dans les espaces intermédiaires (48, 50, 52) à l'intérieur de l'intervalle spécifié par la valeur maximale et la valeur minimale, de sorte que la pression de gaz dans l'espace de stockage de gaz se rapproche au maximum d'une valeur de consigne.

9. Dispositif selon une des revendications précédentes, **caractérisé en ce que** les espaces intermédiaires (48, 50, 52) présentent respectivement au moins un orifice de ventilation à régulation de pression (49, 51, 53).

10. Procédé de régulation d'apport de gaz dans un système de stockage de gaz d'une installation biogaz, dans lequel le système de stockage de gaz comprend au moins deux accumulateurs de gaz commutés en cascade en série qui possèdent respectivement un espace intermédiaire entre une membrane d'accumulateur de gaz et un revêtement de protection, comprenant les étapes suivantes :
- du gaz est amené respectivement aux espaces intermédiaires (48, 50, 52) des accumulateurs de gaz (12, 14, 16),
- la pression de gaz dans les espaces intermédiaires (48, 50, 52) est respectivement mesurée, et
- sur la base de la pression de gaz mesurée dans les espaces intermédiaires (48, 50, 52), la pression de gaz dans les espaces intermédiaires (48, 50, 52) est régulée respectivement par une commande de l'apport de gaz dans les espaces intermédiaires (48, 50, 52).

11. Procédé selon la revendication 10, **caractérisé en ce que** la pression de gaz dans un espace intermédiaire respectif (48, 50, 52) d'un accumulateur de gaz est régulée sur la base d'une valeur de consigne pour la pression de gaz dans l'espace intermédiaire de cet accumulateur de gaz, ainsi que sur la base d'une valeur de consigne pour la pression de gaz dans l'espace intermédiaire d'au moins un autre accumulateur de gaz.

12. Procédé de régulation du transport de gaz dans un système de stockage de gaz d'une installation biogaz, dans lequel le système de stockage de gaz comprend au moins deux accumulateurs de gaz commutés en cascade en série qui possèdent respectivement un espace intermédiaire entre une membrane d'accumulateur de gaz et un revêtement de protection
- des espaces de stockage (42, 44, 46) des accumulateurs de gaz (12, 14, 16) pour le gaz étant reliés respectivement les uns aux autres par au moins une conduite de trop-plein de gaz (60, 62),
- au moins un compresseur à gaz étant disposé dans ladite au moins une conduite de trop-plein de gaz (60, 62), et
- le rendement d'au moins un compresseur à gaz étant régulé de manière à assurer le transport de gaz entre les espaces de stockage (42, 44, 46) via ladite au moins une conduite de trop-plein de gaz (60, 62).

13. Procédé selon la revendication 12, **caractérisé en ce que** la pression de gaz dans les espaces de stockage (42, 44, 46) est mesurée, et **en ce que** le rendement dudit au moins un compresseur à gaz est régulé sur la base de la pression de gaz mesurée d'au moins ces accumulateurs de gaz (12, 14, 16) qui sont reliés par la conduite de trop-plein de gaz (60, 62) munie d'au moins un compresseur à gaz, et/ou **en ce que** le niveau de remplissage de gaz est mesuré dans les espaces de stockage (42, 44, 46) et, **en ce que** le rendement dudit au moins un compresseur à gaz est régulé sur la base du niveau de remplissage de gaz mesuré d'au moins ces accumulateurs de gaz (12, 14, 16) qui sont reliés par la conduite de trop-plein de gaz (60, 62) munie d'au moins un compresseur à gaz.

14. Procédé selon une des revendications 12 ou 13, **caractérisé en ce que** ledit au moins un compresseur à gaz est dimensionné de manière à éviter la survenance d'une surpression ou dépression non autorisée dans un des accumulateurs de gaz même en cas de rendement maximal du compresseur à gaz.

15. Procédé selon une des revendications 10 à 14, **caractérisé en ce que** la pression de gaz (12, 14, 16) dans l'espace de stockage de gaz est mesurée en dessous de la membrane d'accumulateur de gaz, et dans lequel la pression de gaz mesurée dans l'espace de stockage de gaz est prise en compte lors de la régulation de la pression de gaz dans les espaces intermédiaires (48, 50, 52) .

16. Procédé selon la revendication 15, **caractérisé en ce que** l'apport de gaz est commandé de telle sorte que la pression de gaz dans les espaces intermédiaires (48, 50, 52) ne dépasse pas une valeur maximale définie, et/ou **en ce que** l'apport de gaz est commandé de telle sorte que la pression de gaz dans les espaces intermédiaires (48, 50, 52) ne passe pas en dessous d'une valeur minimale définie.

17. Procédé selon la revendication 16, **caractérisé en ce que** la pression de gaz dans les espaces intermédiaires (48, 50, 52) est régulée à l'intérieur de l'intervalle spécifié par la valeur maximale et la valeur minimale de sorte que la pression de gaz dans l'espace de stockage de gaz se rapproche au maximum d'une valeur de consigne.

18. Procédé selon une des revendications 10 à 17, **caractérisé en ce que** les espaces intermédiaires (48, 50, 52) sont ventilés respectivement par au moins un orifice de ventilation à régulation de pression (49, 51, 53) .
